# EUROPEAN PATENT APPLICATION

(11) **EP 4 335 838 A1**
(43) Date of publication of application: **13.03.2024**
(21) Application number: 22194153.7
(22) Date of filing: 06.09.2022
(51) Int. Cl.: C07C 67/03, C07C 69/24, C07C 29/149, C07C 31/125, C07C 41/03, C07C 43/11, C07C 1/24, C07C 11/02

(54) **INSECT OILS AS NATURAL RAW MATERIALS FOR ALCOHOL PRODUCTION**

(71) Applicant: Sasol Chemicals GmbH, 20537 Hamburg (DE)
(72) Inventor: ROMMERSKIRCHEN, Renke, 48301 Nottuln (DE)
(74) Representative: Müller Schupfner & Partner Patent- und Rechtsanwaltspartnerschaft mbB

(57) **Abstract**

Subject matter of the present invention is a method for the production of fatty alcohols from alternative, renewable natural raw materials and the use of the fatty alcohols to produce surfactant intermediates or surfactants. More specifically, the invention describes the transesterification of triglycerides and subsequent hydrogenation to fatty alcohols in an industrially desired carbon range, using insect oils, in particular oils from the black soldier fly larvae, as the feed stream or as part of the feed stream.

## Description

### FIELD OF THE INVENTION

The present invention relates generally to the production of fatty alcohols from alternative, renewable natural raw materials and the use of the fatty alcohols to produce surfactant intermediates or surfactants. More specifically, the invention describes the transesterification of triglycerides and subsequent hydrogenation to fatty alcohols in an industrially desired carbon range, using insect oils, in particular oils from the black soldier fly larvae, as the feed stream or as part of the feed stream.

### BACKGROUND OF THE INVENTION AND DISCUSSION OF THE PRIOR ART

Surfactant intermediates such as alcohol sulfates, alcohol ether carboxylates, alcohol alkoxylates and alcohol ether sulfates, have traditionally been manufactured using conventional feedstocks such as kerosene, petroleum-derived materials and the like. However, there is a pressing need to identify alternative feedstocks not originating from fossil fuels.

Fats and oils containing lipids or triglycerides are known to be important feedstocks for the production of various industrial products. Commonly known vegetable oils such as coconut oil or palm kernel oil, comprising such triglycerides, have been described as useful feedstocks to produce biofuels / biodiesel, as well as other lipid-derived products in the non-food product industry (see e.g. WO2018/085064 A1). Growing these crops for oil production requires however substantial portions of land, often accompanied by undesirable deforestation. In addition, the vegetation needs significant time to reach maturity. Utilisation of these plant-based oils is therefore in general an expensive, less-sustainable way of obtaining such feedstock, with a less favourable life cycle assessment (LCA).

As a result, there is a growing need to provide alternative feedstocks to produce industrially important, lipid-derived products such as esters, alcohols and various other compounds that could find application for instance as surfactant intermediates. The cultivation of insects has been suggested as a viable industry to provide feedstock for both nutritional and various industrial applications, in support of a green and bio-based economy.

These insects and their larvae feed on organic waste such as municipal and other waste materials, in this way already providing significant environmental advantages throughout their short lifetime. Insects require less water and the production of CO₂ emissions required by their breeding is much less than crop cultivation. The main product of this industry, obtained from insect larvae material, is protein meal, mostly used for feed and food applications. Insect larvae comprise, however besides protein, also bio-based oils. These oils are excellent sources for lipids or triglycerides, and it has already been demonstrated that, after extraction and purification, these lipids can successfully be used in cosmetics and personal care product formulations (Insects 2022, 13, 41). Examples include lipids extracted from the larvae of the insect species *Hermetia illucens,* also known as the black soldier fly (BSF), that were successfully applied in formulations for shower gels and soaps.

It was also demonstrated that various industrially useful products such as biodiesel can be produced from the triglycerides obtained from insect oils (see e.g. IN201911037067, KR2021051237).

### OBJECT OF THE PRESENT INVENTION

It is the object of this invention to provide a method for producing fatty alcohols from fatty acid methyl esters obtained from insect oils.

It is another object of this invention to provide a method for producing fatty alcohols of a particular hydrocarbon chain length distribution by transesterification of triglycerides contained in insect oils, followed by hydrogenation.

### SUMMARY OF THE INVENTION

The invention is defined in the appended claims and provides a method that can be utilized to produce fatty alcohols from insect oils or insect oils in combination with other sources. These alcohols may be used for making surfactant intermediates and surfactants, finding application over a wide spectrum of technology areas such as Fabric and Home Care, Personal Care and Cosmetics, agriculture, industrial in general as well as oil and gas recovery areas.

It was surprisingly found that triglycerides obtained from the black soldier fly larvae (BSFL) are excellent feed streams for the high yield production of industrially important C_{12 -} C₁₄ fatty alcohols, specifically finding application in personal care and home care product formulations. The hydrocarbons with chain lengths peaking in the range of C₁₂ to C₁₄ are often economically challenging to obtain in high yields from alternative sources. Especially attractive is the fact that the low yield / lack of lower carbon numbers, namely C₁₀ and below, in the feed stream leads to derived alcohol products without the accompanying odour problem associated with short chain alcohols. This is obviously economically favourable, as no additional fractionation steps are required to remove the undesirable lower carbon alkyl range molecules.

Considering alternative nonionic derivatives from fatty alcohols such as alkoxylates, the higher carbon chain lengths of alcohols lead to lower water solubility. These products typically display lower critical micelle concentration (cmc) values, an indication of desirable higher surface activities and enhanced emulsifying performance.

It is clear that products obtained from insect oils have an attractively low environmental footprint and are sustainable and economical sources for producing hydrocarbon products in a highly desired carbon range, most notably the fatty alcohol products obtained from black soldier fly larvae disclosed herein, as well as the production of surfactants and other products by further derivatization, including their formulations.

### DETAILED DESCRIPTION

In many Home Care, Fabric Care, or Personal Care formulations fatty alcohols and their derivatives like nonionic surfactants (e.g. alkoxylated alcohols) and anionic surfactants (e.g. alcohol ether sulfates, alcohol sulfate, alcohol ether carboxylates), are applied. The largest volume products are based on the so-called mid-cut alcohols which are extensively used in laundry detergents and cleaning products. It comprises alkyl chain lengths mainly in the range of C₁₂ to C₁₆ carbon atoms, with a maximum at C₁₂. Also commonly used even though in considerably smaller volumes are the so-called heavy-cut alcohols with mainly C₁₆ and C₁₈ carbon atoms in the alkyl chain.

**Table 1: Common carbon chain distribution of fatty alcohol compositions used in large-volume consumer products**

| Carbon atoms in the alkyl chain of the alcohol | Mid-cut alcohol [wt%] | Heavy-cut alcohol [wt%] |
|---|---|---|
| C8 | 0-0.5 | - |
| C10 | 0-1.0 | - |
| C12 | 60-75 | 0-1 |
| C14 | 20-30 | 0-2.5 |
| C16 | 2-8 | 25-35 |
| C18 | 0-0.5 | 65-75 |
| C20 | - | 0-2 |

Commonly fatty alcohols in these carbon chain ranges are obtained from natural sources such as palm oil, palm kernel oil, and coconut oil, or manufactured based on petrochemical feed streams.

In addition to various sustainability benefits when using insect oil-derived feedstocks, specifically the black soldier fly larvae, the insect oils potentially provide feedstocks that have very desirable properties for surfactant production, such as carbon distributions in a sought-after carbon number range.

The insect oil-derived feedstocks can either be used alone or blended with various conventional hydrocarbon feedstocks. The feed stream according to the present inventions consist or comprises such insect oil-derived feedstocks.

A method for producing fatty alcohols may include a first step of providing a feed stream comprising fatty acid methyl esters, wherein at least 5 wt% of the fatty methyl esters were obtained from an insect oil, and at least 92 wt% of these fatty acid methyl esters have alkyl chain lengths > C₁₀. At least 20 wt%, preferably at least 40 wt%, more preferably at least 60 wt%, of the fatty acid methyl esters obtained from the insect oil has alkyl chain lengths in the range of C₁₂ to C₁₄. The feed stream is subsequently hydrogenated to form fatty alcohols.

The feed stream preferably comprises at least 80 wt%, more preferably at least 90 wt% and most preferably at least 95 wt% of fatty acid methyl esters.

The invention is further exemplified by the fact that at least 25 wt%, more preferably at least 50 wt%, but most preferably at least 75 wt%, of the fatty methyl esters, were obtained from an insect oil.

According to a preferred embodiment of the invention at least 95 wt%, more preferably at least 97 wt%, of the fatty acid methyl esters have alkyl chain lengths > C₁₀ with the further limitation that at least 40 wt%, more preferably at least 60 wt%, of the fatty acid methyl esters have alkyl chain lengths in the range of C₁₂ to C₁₄.

The method may further include a combination of the feed stream with at least one non-insect derived feed stream comprising fatty acid methyl esters, wherein at least 20 to 60 wt% of the fatty acid methyl esters from the non-insect derived oil have alkyl chain lengths of C₁₂ to C₁₄.

The method to produce fatty alcohols may include a feed stream comprising fatty acid methyl esters obtained from insect oils as defined above and non-insect derived oils, wherein at least 20 wt%, more preferably 40 wt% and most preferably 60 wt%, of the fatty acid methyl esters from the non-insect-derived oil have alkyl chain lengths in the range of C₁₂ to C₁₄.

The method may further include that prior to providing the feed stream comprising fatty acid methyl esters, a first stream is provided comprising triglycerides, wherein at least 5 wt% of the triglycerides are obtained from insects, and the first feed stream is subsequently transesterified to form the feed stream comprising fatty acid methyl esters.

Typically, at least 20 wt%, more preferably at least 40 wt% or most preferably at least 60 wt%, of the triglycerides from the first feed stream - with respect to the acid groups contained of the triglycerides - have alkyl chain lengths in the range of C₁₂ to C₁₄. It is also beneficial for at least 25 wt%, more preferably at least 50 wt% and most preferably 75 wt%, of the triglycerides to have been obtained from insects.

The method may also include the fatty acid methyl esters to be fractionated, for example to form the fatty acid methyl esters obtained from a non-insect derived oil, for example comprising at least 20 wt%, more preferably 40 wt% and most preferably 60 wt%, of the fatty acid methyl esters having an alkyl chain length in the range of C₁₂ to C₁₄ or to form the feed stream comprising fatty acid methyl esters.

Insect oils derived from the black soldier fly larvae (BSFL) form a particularly beneficial feed stream or a particularly beneficial part of the feed stream.

According to one embodiment of the invention the method provides fatty alcohol mixture with at least 20 wt%, more preferably at least 40 wt% or most preferably at least 60 wt%, of linear alcohols with a terminal OH group, with alkyl chain lengths in the range of C₁₂ to C₁₄ and at least 95 wt%, more preferably at least 97 wt%, of linear alcohols with a terminal OH groups, with alkyl chain lengths of > C₁₀.

These fatty alcohols produced as described above may typically be applied to produce surfactant intermediates or surfactants such as, but not limited to alcohol sulfates, alcohol alkoxylates, alcohol ether sulfates and / or alcohol ether carboxylates. Paraffins are also a typical product that may be produced from the fatty alcohols described above.

The invention includes the methods of transforming the fatty alcohols produced as described above, to make products such as:
- paraffins, by further applying a hydrogenation step to the fatty alcohols,
- alcohol alkoxylates, typically ethoxylated and/or propoxylated alcohols by further applying an additional step of alkoxylating the fatty alcohols,
- alcohol sulfates, by sulfating the fatty alcohols,
- alcohol ether sulfates, by first alkoxylating the fatty alcohols, preferably ethoxylating and/or propoxylating, followed by sulfation of the resulting alkoxylated alcohol, and
- alcohol ether carboxylates, by first alkoxylating the fatty alcohols, preferably ethoxylation and/or propoxylation, followed by carboxymethylation of the resulting alkoxylated alcohol.

As used herein the word "insect" can mean the insects, its larvae, or both. Further, as used herein the word "insect oil" refers to the triglyceride compositions obtained from either the insect, the insect larvae, or mixtures thereof. Furthermore, the word "insect oil" could also refer to mixtures of insect oils obtained from different insects. The terms "insect oil" and "insect fat" can be used interchangeably and the term "insect oil" does not necessarily means that the "insect oil" is in a liquid state at room temperature (25°C) and may also be solid / non-flowable at room temperature.

The insect oil-derived fatty alcohols and their derivatives, including but not limited to surfactants (e.g. alkoxylated alcohols, alcohol sulfates, alcohol alkoxy sulfates, ether carboxylates, carboxylated alcohol alkoxylates), esters, and paraffins may be used in detergent compositions, including but not limited to liquid laundry detergents, gel detergents, a single- or multi-phase unit dose detergents, detergent powders, detergent compositions incorporated into fibrous products, in laundry pre-treat products, in fabric softener compositions, in liquid hand dishwashing composition, in solid or liquid automatic dish-washing detergent, in hard surface cleaner, and mixtures thereof. Further use of the above mentioned products can be in various personal care products, for hair care and/or skin care applications, including but not limited to leave-on and rinse-of compositions, sun protection, shampoo, conditioner, shower baths and oils, cosmetics, creams and lotions, lipsticks, deodorants, and antiperspirants. Addition application areas of the above mentioned products can be for industrial use, including but not limited to emulsions, suspensions, and dispersions, firefighting foams, agrochemical formulations, e.g. insecticides, pesticides, and solutions for plant protection, metal working fluids, lubrication fluids, products for the production of oil and gas (including but not limited to enhanced oil recovery formulations, fracking fluids, and foaming, cleaning, and cementing products), paints, inks, and coatings for household or industrial use, additives for paper processing industry, adhesives, and additives for asphalts, amongst others.

Suitable insect oils that can be used to synthesize the described fatty alcohols include, but are not limited to *Hermetial illucens* or the black soldier fly (BSF), more specifically the black soldier fly larvae (BSFL).

### EXPERIMENTAL SECTION

Various oils with varying triglyceride content were used to demonstrate the manufacture of the fatty alcohols and related derivatives, as shown in Table 2.

**Table 2: Fatty acid composition of different sources of triglycerides (determined by GC)**

| **OIL** | ≤ **C8 [%]** | **C10 [%]** | **C12 [%]** | **C14 [%]** | **C16 [%]** | **C18 [%]** | **C20+ [%]** |
|---|---|---|---|---|---|---|---|
| **Insect Oil 1 (BFSL)** | - | 1.1 | 37.2 | 6.4 | 14.9 | 40.4 | - |
| **Coconut Oil** | 7.0 | 5.7 | 46.9 | 19.0 | 9.5 | 11.8 | - |
| **Palm Kernel Oil** | 4.5 | 5.0 | 44.0 | 15.5 | 10.0 | 21.0 | - |

| | | | | | | | |
|---|---|---|---|---|---|---|---|
| Insect Oil 1 (BFSL): Commercially available from Hexafly Coconut Oil: Commercially available from Gustav Heess GmbH Palm Kernel Oil: Commercially available from KLK Oleo, Emmerich, Germany | | | | | | | |

All raw materials were dried under vacuum and stored under molecular sieve 0.3 nm. In addition, a drying tube filled with CaCl₂ was used.

In order to produce the fatty acid methyl esters, a transesterification procedure as outlined below was carried out on the oils shown above in Table 2.

### General Transesterification Procedure:

- The flask was charged with the oil.
- Solid NaOMe (1 wt%) was dissolved in an excess of MeOH (same volume as oil) and added while stirring under N₂ atmosphere.
- The reaction mixture was heated to about 65°C and refluxed for approx. 2.5 h.
- After completion the mixture was cooled to 0°C and quenched with aqueous hydrochloric acid, to reach a pH value of 7.
- The excess of MeOH was removed under reduced pressure.
- To obtain the fatty acid methyl ester with higher purity the resulting product was mixed with the same amount of aqueous NaCl solution (10%). After phase separation the product was obtained as the upper organic phase. Methyl ester compositions obtained for the various feed streams, are shown in Table 3.

**Table 3: Methyl ester composition IO 1-ME: insect oil 1 after transesterification; CO-ME: coconut oil after transesterification**

| **Product** | **≤ C8 [%]** | **C10 [%]** | **C12 [%]** | **C14 [%]** | **C16 [%]** | **C18 [%]** | **C20+ [%]** |
|---|---|---|---|---|---|---|---|
| **IO 1-ME** | - | 1.2 | 37.3 | 6.4 | 13.7 | 41.5 | - |
| **CO-ME** | 7.3 | 6.1 | 47.9 | 18.9 | 9.2 | 10.7 | - |

| | | | | | | | |
|---|---|---|---|---|---|---|---|
| *IO 1* = *Insect Oil 1; ME* = *Methyl ester; CO* = *Coconut oil* | | | | | | | |

Sometimes it is desired to have a composition enriched in a certain carbon chain length. For these cases, it is common practise to fractionate the methyl esters. It is an additional advantage if the amount of unwanted shorter carbon chains (≤ C₁₀) is as low as possible, in order to reduce the energy required for the distillation step, as well as to lower the amount of by-products generated.

### FRACTIONATION

The remaining solution was filtered and fractionally distilled under reduced pressure to obtain the various fractions of the fatty acid methyl esters, with specific conditions depending on their boiling points (*Organikum 23. Auflage, 2009*)*.*

The following fractions were prepared:

**Table 4: Methyl ester compositions obtained after fractionation**

| **COMPOUND** | **Carbon-DISTRIBUTION [wt %]** | | | | | |
|---|---|---|---|---|---|---|
| | **C8 Acid** | **C8 ME** | **C10 Acid** | **C10 ME** | **C12 Acid** | **C12 ME** |
| **Insect Oil 1 (IO 1-ME) (BFSL)** | - | - | - | - | - | 90.5 |
| **Coconut Oil (CO-ME)** | - | 0.02 | - | 3.0 | 0.12 | 88.0 |

| **COMPOUND** | **Carbon-DISTRIBUTION (continued) [wt %]** | | | | | |
|---|---|---|---|---|---|---|
| | **C14 Acid** | **C14ME** | **C16 Acid** | **C16 ME** | **Others** | |
| **Insect Oil 1 (IO 1-ME) (BFSL)** | - | 9.3 | -- | 0.4 | 0.5 | |
| **Coconut Oil (CO-ME)** | - | 8.6 | -- | 0.1 | - | |

The various methyl ester feed streams were subsequently hydrogenated to deliver the fatty alcohols.

### General method of hydrogenation:

In a typical experiment, 5-10 g of a copper-based hydrogenation catalyst was placed into a fixed basket in a 300 ml volume high-pressure autoclave. The catalyst was subsequently reduced under a hydrogen-nitrogen gas-phase at 200°C for 5 hrs.

After cooling the reactor, 50-100 ml of the pure fatty acid methyl ester feed was added under a nitrogen-inert atmosphere to the autoclave. The hydrogenation reaction was performed in a pure hydrogen atmosphere at 220-240°C and 250-300 bar for 5-15 hrs. After cooling down and nitrogen-neutralization, the product was filtered to remove catalyst residues, before analyses by gas chromatograph.

The following results were obtained:

**Table 5: Fatty alcohols produced from the fatty acid methyl esters**

| | **Insect Oil 1 (IO 1-FA) (wt %)** | **Coconut Oil (CO-FA) (wt %)** | **Palm Kernel Oil (PKO-FA) (wt%)** |
|---|---|---|---|
| **Total Fatty Alcohols (FA)** | 98.6 | 98.3 | 99.0 |
| **Total Esters** | 0.9 | 1.5 | 0.7 |
| **Total Paraffins** | 0.4 | 0.2 | 0.2 |
| **Total Ethers** | 0.1 | 0.1 | 0.0 |

**Table 6: Properties of some of the fatty alcohols produced from the methyl esters in Table 5**

| **Property** | **PKO-FA** |
|---|---|
| Density (25°C) [g/cm3] *(ASTM D 7042)* | 0.835 |
| Kin. Viscosity (25°C) [mm2/s] *(ASTM D 7042)* | 21 |
| Dyn. Viscosity (25°C) [mPas] *(ASTM D 7042)* | 17 |
| Density (40°C) [g/cm3] *(ASTM D 7042)* | 0.824 |
| Kin. Viscosity (40°C) [mm2/s] *(ASTM D 7042)* | 12 |
| Dyn. Viscosity (40°C) [mPas] *(ASTM D 7042)* | 10 |
| Boiling point [°C] *(ASTM E 537-12)* | 17-21 |

In addition, alcohol ethoxylates (ethoxylation grade = 9) were prepared from some fatty alcohols produced according to the invention.

### General ethoxylation procedure:

The fatty alcohol obtained from the bio-oil and aqueous KOH (50%) catalyst (0,15w%) was charged to a laboratory ethoxylation reactor. The remaining water was stripped at 120°C, a pressure of 300 mbar and nitrogen flow, for 120 min.

Hereafter, the reaction temperature was increased to 160°C and nitrogen added. The calculated amount of ethyleneoxide (EO) was added continuously till all EO was added. After the complete addition of EO, the mixture was allowed to boil down till the pressure remained constant at 160°C. Afterwards, free EO was removed under reduced pressure at 80°C for 90 min., 50 mbar and nitrogen flow. The product was drained from the reactor.

The following properties of specific alkoxylated alcohols were determined and shown in Table 7.

**Table 7: Properties of some ethoxylated alcohols (ethoxylation grade = 9)**

| **Property** | | **PKO-AE9** | | | | | **IO 1-AE9** | | | |
|---|---|---|---|---|---|---|---|---|---|---|
| Cloud point [°C] 10% in 25BDG solution *(DIN EN 1890)* | | 83.5 | | | | | 83.4 | | | |
| Surface tension [mN/m] @ 25°C *(DIN EN 14370)* | | 31.3 | | | | | 30.5 | | | |
| Wetting @ 25°C [s] | | Cotton disc: 45 | | | | | Cotton disc: 41 | | | |
| 1 q/L *(DIN EN 1772)* | | Draves test: 28 | | | | | Draves test: 20 | | | |

| **Product** | **Surfactant concentration [10%]** | | | | | | | | | |
|---|---|---|---|---|---|---|---|---|---|---|
| | 10 | 20 | 30 | 40 | 50 | 60 | 70 | 80 | 90 | 100 |
| **PKO-AE9** | ○ | ○ | ∘ | • | • | • | • | • | ○ | ◊ |
| **IO 1-AE9** | ○ | ○ | ∘ | ○ | • | • | ○ | ∘ | ○ | ◊ |
| | Explanation: • = gel phase; ∘ = clear liquid; ◊ = cloudy liquid | | | | | | | | | |

The ethoxylated alcohols prepared from the insect oil (IO 1-AE9) displayed enhanced wetting behaviour, as well as less gel formation when compared to similar products prepared from palm kernel oil (PKO-AE9).

Further derivatives from the alcohols such as alcohol sulfates and paraffins as well as from the alkoxylated alcohols such as alcohol ether sulfates and alcohol ether carboxylates were prepared according to the following general methods:

### General procedures to produce further derivatives:

### General procedure for the dehydration of the fatty alcohols to form paraffins:

The specific conditions will depend on the fatty alcohol mixture to be dehydrated. A typical method is described in US10,654,765B2, where 2474g of 1-hexadecanol (NACOL 16) are mixed with 500g Al₂O₃ and a solvent such as xylene, in a flask equipped with a water separator. The flask is heated to 295°C for 4.5 hours. The linear olefin, hexadecane, was distilled under vacuum. The product is a mixture of alpha- and internal olefins.

In order to produce the paraffinic product, the olefins produced above are hydrogenated. According to state of the art hydrogenation methods, 685 g of the hexadecane obtained above is hydrogenated for 7 hours at 98°C, over a heterogeneous Ni-containing catalyst at 20 bar H₂ pressure and filtrated after cooling.

### General procedure for sulfation of the fatty alcohols / ethoxylated alcohols:

The sulfation of the alkoxylated alcohol / alcohol is typically carried out in a continuous falling film reactor maintained at a temperature from 45°C to 75°C using sulfur trioxide which is diluted with air (e.g. 3-7% SO₃ mole fraction in process air) as the sulfating agent. The mole ratio of SO₃ to feedstock is typically maintained at a range of from 0.8 to 1.2:1. The resulting sulfated products is transferred into a neutralization cycle and neutralized with an organic or inorganic base at 30°C - 60°C. A solvent or water can be added during the neutralization step to reduce the viscosity during mixing/neutralization.
(Organic Process Research & Development 1998, 2, 194-202)

### General procedure for the carboxymethylation of the alkoxylated alcohols:

The ethoxylate is placed in the flask and heated to 80°C under vacuum, 20 mbar. NaOH (50%, aqueous, 2.1 eq.) and monochloroacetic acid (80%, aqueous, 1 eq) are slowly added via metering pumps. The acid and base are added simultaneously. The resulting reaction water plus the water from the acid/base are removed under reduced pressure (20 mbar). The dosing time takes approx. 2.5 hrs. After addition, the mixture is stirred for 3 h at 80 °C and 20 mbar. Afterwards the reaction mixture is adjusted at 80°C with 10% H₂SO₄ to a pH value of 1-2 and stirred for approx. 1 hr. It is transferred into a heatable separating funnel (80°C) and the organic and water phase are separated.

## Claims

1. A method for producing fatty alcohols comprising the following steps:
i) providing a feed stream comprising fatty acid methyl esters, wherein
a) at least 5 wt% of the fatty acid methyl esters were obtained from an insect oil,
b) at least 92 wt% of the fatty acid methyl esters obtained from the insect oil have alkyl chain lengths > C10, and
c) at least 20 wt% of the fatty acid methyl esters obtained from the insect oil have alkyl chain lengths in the range of C12 to C14,
ii) hydrogenating the fatty acid methyl esters of step i) to form fatty alcohols.

2. The method according to claim 1, wherein the feed stream comprises at least 80 wt%, more preferably at least 90 wt% and most preferably at least 95 wt%, of fatty acid methyl esters.

3. The method according to at least one of the preceding claims, wherein at least 25 wt%, more preferably at least 50 wt%, most preferably at least 75 wt%, of the fatty acid methyl esters were obtained from the insect oil.

4. The method according to at least one of the preceding claims, wherein
i) at least 95 wt%, more preferably at least 97 wt%, of the fatty acid methyl esters have alkyl chain lengths > C10, and
ii) at least 40 wt%, more preferably at least 60 wt%, of the fatty acid methyl esters have alkyl chain lengths in the range of C12 to C14.

5. The method according to at least one of the preceding claims, wherein the feed stream comprises fatty acid methyl esters obtained from the insect oil and fatty acid methyl esters obtained from a non-insect derived oil, wherein at least 20 wt%, preferably at least 40 wt%, most preferably at least 60 wt%, of the fatty acid methyl esters from the non-insect derived oil have alkyl chain lengths in the range of C12 to C14.

6. The method according at least one of the preceding claims wherein prior to providing the feed stream comprising fatty acid methyl esters,
i) a first feed stream comprising triglycerides is provided, wherein at least 5 wt% of the triglycerides are obtained from insects,
ii) the first feed stream is trans-esterified to form the feed stream comprising fatty acid methyl esters.

7. The method according to claim 6 wherein the triglycerides from the first feed stream comprise at least 20 wt% of acid groups having alkyl chain lengths in the range of C12 to C14.

8. The method according to at least one of claims 6 or 7 wherein the triglycerides from the first feed stream comprise at least 40 wt%, more preferably at least 60 wt%, of acid groups having alkyl chain lengths in the range of C12 to C14.

9. The method according to at least one of claims 6 to 8, wherein at least 25 wt%, more preferably at least 50 wt%, most preferably at least 75 wt%, of the triglycerides from the first feed stream were obtained from insects.

10. The method according to at least one of the preceding claims, wherein the fatty acid methyl esters are fractionated.

11. The method according to claim 10, wherein the fatty acid methyl esters are fractionated to comprise a stream wherein at least 20 wt% of the fatty acid methyl esters, preferably at least 40 wt %, more preferably at least 60 wt% and most preferably at least 80%, have alkyl chain lengths in the range of C12 to C14.

12. The method according to at least one of the preceding claims, wherein the insect oil is derived from the black soldier fly larvae or the insects are black soldier fly larvae.

13. The use of the fatty alcohols produced by the method of at least one of the preceding claims, to produce surfactant intermediates or surfactants.

14. The use of the fatty alcohols of claim 13 wherein the surfactant intermediates or the surfactants are selected from paraffins, alcohol sulfates, alcohol alkoxylates, alcohol ether sulfates, alcohol ether carboxylates and mixtures thereof.

15. The method of transforming the fatty alcohols produced by the method of at least one of claims 1 to 11 into
a) paraffins, by further applying a hydrogenation step to the fatty alcohols,
b) alcohol alkoxylates, preferably ethoxylated and/or propoxylated alcohols by further applying an additional step of alkoxylating the fatty alcohols,
c) alcohol sulfates, by sulfating the fatty alcohols,
d) alcohol ether sulfates, by first alkoxylating the fatty alcohols, preferably ethoxylating and/or propoxylating the fatty alcohols, followed by sulfation of the resulting alkoxylated alcohol,
e) alcohol ether carboxylates, by first alkoxylating the fatty alcohols, preferably ethoxylating and/or propoxylating the fatty alcohols, followed by carboxymethylation of the resulting alkoxylated alcohol.
